Europäisches Patentamt

European Patent Office

Office européen des brevets

(11) Veröffentlichungsnummer: **0 234 295 B1**

(12) **EUROPÄISCHE PATENTSCHRIFT**

(45) Veröffentlichungstag der Patentschrift: **11.12.91**

(51) Int. Cl.⁵: **C07D 201/08, B01J 23/78, B01J 25/00**

(21) Anmeldenummer: 87100964.3

(22) Anmeldetag: 23.01.87

(54) **Verfahren zur Herstellung von Epsilon-Caprolactam.**

(30) Priorität: 28.01.86 DE 3602376

(43) Veröffentlichungstag der Anmeldung:
02.09.87 Patentblatt 87/36

(45) Bekanntmachung des Hinweises auf die
Patenterteilung:
11.12.91 Patentblatt 91/50

(84) Benannte Vertragsstaaten:
**BE CH DE FR GB IT LI NL**

(56) Entgegenhaltungen:
DE-C- 954 442
GB-A- 1 191 539

(73) Patentinhaber: **BASF Aktiengesellschaft
Carl-Bosch-Strasse 38
W-6700 Ludwigshafen(DE)**

(72) Erfinder: **Hutmacher, Hans-Martin, Dr.
Rüdigerstrasse 70
W-6700 Ludwigshafen(DE)**
Erfinder: **Merger, Franz, Dr.
Max-Slevogt-Strasse 25
W-6710 Frankenthal(DE)**
Erfinder: **Broecker, Franz Josef, Dr.**
**Schwanthaler Allee 20
W-6700 Ludwigshafen(DE)**
Erfinder: **Fischer, Rolf, Dr.
Bergstrasse 98
W-6900 Heidelberg(DE)**
Erfinder: **Vagt, Uwe, Dr.,
Paul-Neumann-Strasse 44
W-6720 Speyer(DE)**
Erfinder: **Harder, Wolfgang, Dr.
Bergwaldstrasse 16
W-6940 Weinheim(DE)**
Erfinder: **Priester, Claus-Ulrich, Dr.
Wiesenstrasse 18
W-6701 Meckenheim(DE)**
Erfinder: **Schneider, Heinz-Walter, Dr.
Brüsseler Ring 43
W-6700 Ludwigshafen(DE)**
Erfinder: **Richter, Wolfgang, Dr.
Am Hüttenwingert 16
W-6706 Wachenheim(DE)**

## Beschreibung

Gegenstand der Erfindung ist ein Verfahren zur Herstellung von ε-Caprolactam aus 5-Formylvaleriansäureestern.

In der GB-PS 1 191 539 wird ein Verfahren zur Herstellung von ε-Caprolactam beschrieben, bei dem man 5-Formylvaleriansäureester mit Wasserstoff, Ammoniak und Wasserdampf in der Gasphase bei 260°C unter Mitverwendung von Kupferkatalysatoren umsetzt. Schwierigkeiten bestehen jedoch hinsichtlich der schlechten Verdampfbarkeit des thermolabilen 5-Formylvaleriansäureesters und den ungenügenden Katalysatorstandzeiten. Aus der japanischen Patentveröffentlichung 29148/1968 ist auch bekannt, daß man 5-Formylvaleriansäureester mit Ammoniak in Gegenwart von Wasser bei einer Temperatur von 230°C und unter einem Druck von 150 bar sowie in Gegenwart von Raney-Nickel in flüssiger Phase umsetzt. Dieses Verfahren hat den Nachteil, daß es bei der technischen Ausführung mit sehr schwankenden Ausbeuten verläuft. Aus der DE-C- 952 442 ist bekannt, daß man durch Umsetzen von Formylvaleriansäureestern mit Ammoniak und Wasserstoff in Gegenwart von Raney-Kobalt und wasserfreiem Dioxan Aminocapronsäureester mit einer Ausbeute von 24 % und Caprolactam mit einer Ausbeute von 40 % erhält.

Es war deshalb die technische Aufgabe gestellt, ein Verfahren zur Herstellung von ε-Caprolactam, ausgehend von 5-Formylvaleriansäureestern zur Verfügung zu stellen, das mit hohen Ausbeuten verläuft und bei dem möglichst wenig Nebenprodukt gebildet werden.

Diese Aufgabe wird gelöst in einem Verfahren zur Herstellung von ε-Caprolactam durch Umsetzen von 5-Formylvaleriansäureestern mit Ammoniak im Überschuß und Wasserstoff in Gegenwart von Hydrierkatalysatoren unter Mitverwendung von Lösungsmitteln bei erhöhter Temperatur unter erhöhtem Druck in flüssiger Phase, dadurch gekennzeichnet, daß man

a) 5-Formylvaleriansäureester mit überschüssigem Ammoniak unter Mitverwendung von $C_1$-$C_4$-Alkanolen als Lösungsmittel in Gegenwart von Kobalt- oder Nickelkatalysatoren unter einem Wasserstoffpartialdruck von 5 bis 1000 bar in flüssiger Phase bei einer Temperatur von 40 bis 130°C umsetzt,

b) aus dem Reaktionsgemisch Ammoniak bis auf einen Gehalt von 0,1 bis 2 Gew.-% und Wasserstoff abtrennt und

c) das so erhaltene Reaktionsgemisch auf eine Temperatur von 150 bis 250°C erhitzt und ε-Caprolactam gewinnt.

Das neue Verfahren hat den Vorteil, daß es mit hohen Ausbeuten verläuft und wenig Nebenprodukte gebildet werden.

Das neue Verfahren ist insofern bemerkenswert, als ausweislich der japanischen Patentveröffentlichung 29148/1968 bei der Verwendung von Lösungsmitteln wie Dioxan anstatt Wasser erhebliche Mengen an Nebenprodukten gebildet werden und die Ausbeute beträchtlich absinkt.

Bevorzugte 5-Formylvaleriansäureester sind 5-Formylvaleriansäurealkylester, insbesondere solche von $C_1$- bis $C_4$-Alkanolen wie Methyl-, Ethyl-, Propyl-, Isopropyl- oder n-Butylester. Geeignete Ausgangsverbindungen sind demnach 5-Formylvaleriansäuremethylester, 5-Formylvaleriansäureethylester, 5-Formylvaleriansäurepropylester, 5-Formylvaleriansäureisopropylester oder 5-Formylvaleriansäure-n-butylester. Besondere technische Bedeutung hat 5-Formylvaleriansäuremethylester erlangt.

Die Umsetzung in der Stufe a) wird unter Mitverwendung von $C_1$ bis $C_4$-Alkanolen als Lösungsmittel durchgeführt. Vorteilhaft verwendet man Alkanole, die der Alkoholkomponente des 5-Formylvaleriansäureesters entsprechen. Demzufolge sind bevorzugte Lösungsmittel Methanol, Ethanol, Propanol, Isopropanol oder n-Butanol. Besonders bevorzugt ist die Kombination 5-Formylvaleriansäuremethylester/Methanol. Vorteilhaft werden die 5-Formylvaleriansäureester als 1 bis 50 gew.-%ige, vorteilhaft 2 bis 35 gew.-%ige, insbesondere 5 bis 25 gew.-%ige Lösung in den genannten Lösungsmitteln eingesetzt.

Bei der Umsetzung wendet man Ammoniak im Überschuß an. Vorteilhaft verwendet man je Mol 5-Formylvaleriansäureester, 2 bis 50 Mol Ammoniak. Besonders gute Ergebnisse erhält man, wenn man je Mol 5-Formylvaleriansäureester 5 bis 30 Mol, insbesondere 10 bis 25 Mol, Ammoniak anwendet.

Die Umsetzung wird in flüssiger Phase bei einer Temperatur von 40 bis 130°C, vorzugsweise von 40 bis 95°C, insbesondere 60 bis 90°C durchgeführt.

Vorteilhaft wendet man je Mol 5-Formylvaleriansäureester 1 bis 20 Mol Wasserstoff an. Man hält einen Wasserstoffpartialdruck von 5 bis 1000 bar, vorzugsweise 20 bis 500 bar, insbesondere 50 bis 200 bar, aufrecht.

Die Umsetzung in Stufe a) wird im Gegenwart von Nickel- oder Kobalt-Katalysatoren durchgeführt. Die Katalysatormetalle können als Vollkatalysatoren, z.B. in feiner Verteilung wie Raney-Nickel oder Raney-Kobalt in Suspensionsfahrweise oder in magnetischer Fixierung, als Mischkatalysatoren oder auf Trägern niedergeschlagen angewandt werden. Geeignete Träger sind beispielsweise Aluminiumoxid, Kieselgel oder Magnesiumsilikate.

Gut geeignet sind auch Skelett-Katalysatoren. Die katalytisch aktiven Metalle werden besonders vorteilhaft in feiner Verteilung angewandt. Deshalb haben sich Skelett-Katalysatoren als besonders geeignet erwiesen.

Besonders bevorzugt wendet man Katalysatoren, die durch Kalzinieren von Verbindungen der Formel I

$$[(Mg_aNi(II)_bCo(II)_cAl_2]CO_3(OH)_{16} \times 4\ H_2O \qquad I$$

in der a für ganze oder Dezimalzahlen von O bis 4 steht und b und c für ganze Zahlen oder Dezimalzahlen von O bis 6 stehen, mit der Maßgabe, daß 2 (a + b + c) = 12 ist, bei einer Temperatur von 200 bis 600°C und anschließende Reduktion mit Wasserstoff bei erhöhter Temperatur, z.B. von 350 bis 400°C, hergestellt wurden. Besonders bewährt haben sich Katalysatoren, die erhalten wurden durch Kalzinieren und Reduzieren von Verbindungen der folgenden Formeln

$$Ni_6Al_2(OH)_{16}CO \times 4\ H_2O$$
$$Ni_5MgAl_2(OH)_{16}CO_3 \times 4\ H_2O$$
$$Co_6Al_2(OH)_{16}CO_3 \times 4\ H_2O$$
$$Co_5MgAl_2(OH)_{16}CO_3 \times 4\ H_2O.$$

Die Verbindungen der Formel I erhält man beispielsweise wie folgt: Nickel, Aluminium, Kobalt und Magnesium werden in Form ihrer wasserlöslichen Salze wie Chloride, Sulfate oder vorzugsweise Nitrate, gemeinsam in Wasser gelöst und zwar in einem Mengenverhältnis, das der gewünschten Zusammensetzung des Katalysators möglichst nahe kommt und in seiner Stöchiometrie der Formel I gehorcht.

Die Metallsalzlösung soll insgesamt etwa 0,5 bis 3 molar, vorzugsweise 1,0 bis 2 molar an Metallionen sein. Sie wird auf Temperaturen von 50 bis 100°C, vorzugsweise bis 100°C, erhitzt und innerhalb von 0,5 bis 10, vorzugsweise bis 3 Minuten, mit einer äquivalenten Menge oder bevorzugt einem geringen Überschuß einer auf 50 bis 100°C, vorzugsweise 80 bis 100°C erhitzten, 1 bis 3, vorzugsweise 1,5 bis 2,5 molaren Lösung eines Alkalidicarbonats vereinigt. Vorteilhaft arbeitet man mit einem Überschuß an Alkalibicarbonat, der bis zu 20 Gew.-%, vorzugsweise 0,5 bis 3 Gew.-%, bezogen auf die theoretische Menge des Bicarbonats, beträgt. Nach der Zugabe der Metallsalzlösung wird zweckmäßig noch 10 bis 30 Minuten, vorzugsweise 15 bis 25 Minuten gerührt und dann der entstehende Niederschlag abfiltriert, mit Wasser gewaschen und bei einer Temperatur von 50 bis 200°C, vorzugsweise 100 bis 160°C getrocknet. Die basischen Carbonate werden in nahezu quantitativen Ausbeuten erhalten. Als Alkalibicarbonate kommen insbesondere Natriumbicarbonat oder Kaliumbicarbonat in Betracht. Es ist aber auch möglich, Ammoniumbicarbonat bei der Fällung zu verwenden. Selbstverständlich können auch Mischungen der genannten Bicarbonate verwendet werden. Außerdem ist es möglich, die Fällung der Metallionen mit Lösungen von Alkalicarbonaten wie Natriumcarbonat und/oder Kaliumcarbonat vorzunehmen, wenn man während des Fällens Kohlendioxid in die die vorgelegte Alkalicarbonatlösung einleitet, was aber im Endeffekt auf eine Fällung mit Bicarbonat hinausläuft. Vorteilhaft wendet man beim Kalzinieren eine Temperatur von 250 bis 400°C für eine Dauer von z.B. 5 bis 40, insbesondere 15 bis 30 Stunden an. Vor der eigentlichen Verwendung des Katalysators wird dieser mit Wasserstoff vorteilhaft bei einer Temperatur von 180 bis 500°C, vorzugsweise 250 bis 450°C, innerhalb von 5 bis 100 Stunden, vorteilhaft 10 bis 25 Stunden, reduziert.

Andere bevorzugte Katalysatoren sind Nickelkatalysatoren, die Nickel in fein verteilter Form auf Träger, insbesonder Magnesiumsilikat, aufgebracht enthalten. Vorteilhaft haben solche Katalysatoren einen Gehalt an Nickel von 30 bis 60 Gew.-%, bezogen auf die gesamte Katalysatormasse einschließlich Träger. Solche Katalysatoren werden beispielsweise in der DE-PS 15 45 428 beschrieben.

Bevorzugt wird Raney-Nickel oder Raney-Kobalt als Katalysator verwendet, wobei man in Suspensionsfahrweise arbeitet, oder den Katalysator in der Reaktionszone an Permanentmagneten oder an Weicheisenkörpern in einem magnetischen bzw. elektromagnetischen Feld fixiert. Solche Magnete sind z.B. in Form von Stäben in der Reaktionszone angeordnet.

Für die Umsetzung hat es sich weiterhin als vorteilhaft erwiesen, eine Verweilzeit von 1 bis 30 Minuten sowie Katalysatorbelastungen von 0,2 bis 2,0 kg 5-Formylvaleriansäureester je Liter Katalysator und Stunde einzuhalten.

Die Umsetzung kann diskontinuierlich, z.B. in einem Hochdruckgefäß, durchgeführt werden. Vorzugsweise führt man die Umsetzung kontinuierlich, z.B. in druckfesten Rührbehältern, z.B. einer Rührbehälterkaskade durch. Es hat sich als vorteilhaft erwiesen, eine Rückvermischung während der Umsetzung zu vermeiden. Besonders bewährt haben sich deshalb Rohrreaktoren, in denen man die alkoholische Lösung von 5-Formylvaleriansäureester und Ammoniak über ein fest angeordnetes Katalysatorbett leitet. Besonders bewährt hat sich hierbei die Sumpffahrweise.

Als Reaktionsaustrag aus der Stufe a erhält man nach dem Entspannen, bei dem der Wasserstoff entfernt wird, Gemische aus 6-Aminocarbonsäureestern, dem mitverwendeten Alkanol, überschüssigem Ammoniak und untergeordneten Mengen an $\epsilon$-Caprolactam.

Bei der Verwendung von 5-Formylvaleriansäuremethylester und Methanol als Lösungsmittel erhält man ein Reaktionsgemisch aus 6-Aminocapronsäuremethylester in Methanol mit einem Anteil von 1 bis 10 Mol.-% an Caprolactam, bezogen auf 6-Aminocapronsäuremethylester sowie Ammoniak.

In einer zweiten Stufe b wird das überschüssige Ammoniak aus dem Reaktionsgemisch entfernt. Dies erfolgt beispielsweise durch Abdestillieren oder durch Strippen mit Inertgas. Die anfallenden Mengen an Ammoniak sowie überschüsssiger Wasserstoff werden vorteilhaft wieder in die Stufe a zurückgeführt. Hierbei hält man einen Ammoniakgehalt in der der weiter zu verwendenden Lösung von 0,1 bis 2 Gew.-%, insbesondere 0,1 bis 1 Gew.-%, ein.

Das so erhaltene von Ammoniak befreite Reaktionsgemisch wird in der nachfolgenden Stufe c auf eine Temperatur von 150 bis 250°C, bevorzugt 170 bis 230°C erhitzt. Zweckmäßig hält man einen Druck ein, der größer oder gleich dem sich einstellenden Druck des Reaktionsgemisches bei der angewandten Temperatur ist. Vorteilhaft hält man Verweilzeiten von 0,5 bis 5 Stunden je nach Art des angewandten Esters und den Reaktionstemperaturen ein.

Die Umsetzung kann diskontinuierlich oder bevorzugt kontinuierlich, z.B. in Hochdruckgefäßen in Kaskadenart angeordnet sein können oder in Rohrreaktoren durchgeführt werden.

Aus der so erhaltenen alkoholischen Lösung von Caprolactam kann dieses in üblicher Weise, z.B. durch Destillation oder Extraktion abgetrennt werden. Die als Lösungsmittel verwendeten Alkanole und gegebenenfalls vorhandener nicht umgesetzter 6-Aminocapronsäureester können wieder in die Stufe a oder c zurückgeführt werden.

Caprolactam wird für die Herstellung von Polyamid-6 verwendet.

Das Verfahren nach der Erfindung sei im folgenden Beispiel veranschaulicht.

Beispiel 1

Stufe a):

Ein vertikal angeordneter Rohrreaktor (Durchmesser 16 mm, Füllhöhe 25 cm, ölbeheizter Doppelmantel) wurde mit 50 ml eines Nickelkatalysators mit 55 Gew.-% Nickeloxid in feiner Verteilung auf Magnesiumsilikat in Form von Strängen von 1,5 mm Durchmesser gefüllt. Der Katalysator wurde in 18 Stunden unter schrittweisem Anheben der Temperatur von 60 auf 330°C und unter Steigern des Wasserstoffgehalts in dem zur Reduktion benutzten Stickstoff-Wasserstoff-Gemisch von 5 auf 50 % reduziert.

Danach wurden unter gleichzeitigem Durchleiten von Wasserstoff bei 80°C und 100 bar stündlich von unten nach oben 99,5 g einer 10,0 gew.-%igen methanolischen 5-Formylvaleriansäuremethylester-Lösung und 16,2 g flüssiges Ammoniak durch den Reaktor gepumpt. Vom Kopf des Reaktors gelangte das Reaktionsgemisch über einen Kühler in einen Abscheider, aus dem der Reaktionsaustrag sowie stündlich 25 l Abgas ausgeschleust wurden.

Stufe b):

Der Reaktionsaustrag wurde kontinuierlich auf den Kopf einer auf 40°C beheizten, 40 cm langen und mit V$_2$A-Maschendrahtringen (ø 3 mm) gefüllten Kolonne (Stripper) geleitet, durch die im Gegenstrom stündlich 22 l Stickstoff geblasen wurden. Am Kolonnensumpf wurden stündlich 91,2 g gestrippter Reaktionsaustrag mit 0,5 Gew.-% Ammoniak erhalten, der nach quantitativer gaschromatographischer Analyse 9,43 % 6-Aminocapronsäuremethylester und 0,36 % Caprolactam enthielt, entsprechend Ausbeuten von 86,0 % 6-Aminocapronsäuremethylester und 4,2 % Caprolactam bezogen auf eingesetzten 5-Formylvaleriansäuremethylester.

Stufe c):

Ein Teilstrom dieses Austrags von stündlich 18,3 g wurde kontinuierlich bei 105 bar durch einen auf 225°C temperierten Rohrreaktor von 8,71 m Länge und 3,17 mm Durchmesser (Rohrschlange) gepumpt. Am Reaktorausgang wurde das Reaktionsgemisch auf Raumtemperatur abgekühlt und auf Normaldruck entspannt und enthielt danach nach quantitativer gaschromatographischer Analyse 6,47 % Caprolactam und 0,78 % 6-Aminocapronsäuremethylester. Dies entspricht Ausbeuten von 75,6 % Caprolactam und 7,1 % 6-Aminocapronsäuremethylester, bezogen auf den in die Hydrierstufe eingesetzten 5-Formylvaleriansäuremethylester.

Beispiel 2

In einem vertikalen Rohrreaktor (Durchmesser: 14 mm, Länge: 450 mm) ist ein Stab mit einem Durchmesser von 9 mm zentral angeordnet, auf dem Permanentmagnete (mit der Feldstärke 500 A/m) befestigt sind. Die Magnete werden mit 11,0 g Raney-Nickel beladen, indem man eine Suspension von Raney-Nickel in Wasser von unten nach oben durch den Reaktor leitet.

Danach werden unter gleichzeitigem Durchleiten von 8,7 l Wasserstoff bei 76°C und 80 bar stündlich von unten nach oben 77,3 g einer 12,0 g gew.-%igen methanolischen 5-Formylvaleriansäuremethylester-Lösung und 24 ml flüssiges Ammoniak durch den Reaktor gepumpt. Vom Kopf des Reaktors gelangt das Reaktionsgemisch über ein Konstanthaltungsventil auf den Kopf

einer auf 40°C beheizten, 40 cm langen und mit V₂A-Machendrahtringen (ø 3 mm) gefüllten Kolonne (Stufe 3) geleitet, durch die im Gegenstrom stündlich 22 l Stickstoff geblasen wurden. Am Kolonnensumpf wurden stündlich 72,3 g gestrippter Reaktionsaustrag mit 0,5 Gew.-% Ammoniak erhalten, der nach quantitativer gaschromatographischer Analyse 9,35 % 6-Aminocapronsäuremethylester und 0,25 % Caprolactam enthielt, entsprechend Ausbeuten von 89,1 % 6-Aminocapronsäuremethylester und 3,1 % Caprolactam bezogen auf eingesetzten 5-Formylvaleriansäuremethylester.

Die Stufe c wird entsprechend Beispiel 1 durchgeführt. Die Ausbeute am Caprolactam beträgt 78 %.

**Patentansprüche**

1. Verfahren zur Herstellung von ε-Caprolactam durch Umsetzen von 5-Formylvaleriansäureestern mit überschüssigem Ammoniak und Wasserstoff in Gegenwart von Hydrierkatalysatoren unter Mitverwendung eines Lösungsmittels bei erhöhter Temperatur und unter erhöhtem Druck in flüssiger Phase, dadurch gekennzeichnet, daß man

   a) 5-Formylvaleriansäureester mit überschüssigem Ammoniak unter Mitverwendung von C₁-C₄-Alkanolen als Lösungsmittel in Gegenwart von Kobalt- oder Nickelkatalysatoren unter einem Wasserstoffpartialdruck von 5 bis 1000 bar bei 40 bis 130°C umsetzt,
   b) aus dem erhaltenen Reaktionsgemisch Ammoniak bis auf einen Gehalt von 0,1 bis 2 Gew.-% und Wasserstoff entfernt und
   c) das so erhaltene Reaktionsgemisch auf eine Temperatur von 150 bis 250°C erhitzt und anschließend Caprolactam gewinnt.

2. Verfahren nach Anspruch 1, dadurch gekennzeichnet, daß man Katalysatoren verwendet, die durch Kalzinieren von Verbindungen der Formel I

   $$[(Mg_aNi(II)_bCo(II)_c)Al_2]CO_3(OH)_{16} \times 4\ H_2O$$

   in der a für ganze oder Dezimalzahlen von 0 bis 4 steht und b und c für ganze oder Dezimalzahlen von 0 bis 6 stehen, mit der Maßgabe, daß 2 x (a + b + c) = 12 ist, bei Temperaturen von 200 bis 600°C und anschließende Reduktion mit Wasserstoff bei erhöhter Temperatur hergestellt worden sind.

3. Verfahren nach Anspruch 1, dadurch gekennzeichnet, daß man Nickel-Katalysatoren verwendet, die 30 bis 60 Gew.-% Nickel in fein verteilter Form auf Magnesiumsilikat niedergeschlagen enthalten.

4. Verfahren nach den Ansprüchen 1 bis 3, dadurch gekennzeichnet, daß man 5-Formylvaleriansäureester in Lösung mit Alkanolen und Ammoniak in Sumpffahrweise über ein fest angeordnetes Katalysatorbett leitet.

5. Verfahren nach Anspruch 1, dadurch gekennzeichnet, daß man Raney-Nickel oder Raney-Kobalt in Suspensionsfahrweise verwendet.

6. Verfahren nach den Ansprüchen 1 und 5, dadurch gekennzeichnet, daß man das Raney-Nickel oder Raney-Kobalt in der Reaktionszone magnetisch oder elektromagnetisch fixiert.

7. Verfahren nach den Ansprüchen 1 bis 6, dadurch gekennzeichnet, daß man in Stufe a eine Verweilzeit von 1 bis 30 Minuten einhält.

8. Verfahren nach den Ansprüchen 1 bis 7, dadurch gekennzeichnet, daß man eine Katalysatorbelastung von 0,2 bis 2,0 kg 5-Formylvaleriansäureester je Liter Katalysator und Stunde einhält.

9. Verfahren nach den Ansprüchen 1 bis 8, dadurch gekennzeichnet, daß man 5-Formylvaleriansäuremethylester, gelöst in Methanol, verwendet.

**Claims**

1. A process for the preparation of ε-caprolactam by reacting the 5-formylvalerate with excess ammonia and hydrogen in the presence of hydrogenation catalysts and of a solvent at elevated temperatures and under super-atmospheric pressure in the liquid phase, wherein
   a) a 5-formylvalerate is reacted with excess ammonia in the presence of a C₁-C₄-alkanol as a solvent and in the presence of a cobalt or nickel catalyst under a hydrogen partial pressure of 5 to 1000 bar at from 40 to 130°C,
   b) excess ammonia up to a content of from 0.1 to 2% by weight and hydrogen are separated off from the reaction mixture and
   c) the reaction mixture thus obtained is heated to 150-250°C and the ε-caprolactam is obtained.

2. A process as claimed in claim 1, wherein the catalyst used is prepared by calcining a compound of the formula I

$$[(Mg_aNi(II)_bCo(II)_c)Al_2]CO_3(OH)_{16} \times 4\ H_2O$$

where a is an integer or decimal number from 0 to 4 and b and c are each an integer or decimal number from 0 to 6, with the proviso that 2 (a + b + c) = 12, at from 200 to 600°C and then reducing the product with hydrogen at elevated temperatures.

3. A process as claimed in claim 1, wherein a nickel catalyst which contains from 30 to 60% by weight of nickel deposited in finely divided form or magnesium silicate is used.

4. A process as claimed in any of claims 1 to 3, wherein the 5-formylvalerate in solution with an alkanol and ammonia is passed over a fixed bed catalyst by the liquid phase procedure.

5. A process as claimed in claim 1, wherein Raney nickel or Raney cobalt is used in a liquid phase procedure.

6. A process as claimed in either of claims 1 and 5, wherein Raney nickel or Raney cobalt is fixed magnetically or electromagnetically in the reaction zone.

7. A process as claimed in any of claims 1 to 6, wherein a residence time of 1 to 30 minutes is maintained in stage a.

8. A process as claimed in any of claims 1 to 7, wherein the space velocity maintained is from 0.2 to 2.0 kg of 5-formylvalerate per liter of catalyst per hour.

9. A process as claimed in any of claims 1 to 8, wherein methyl 5-formylvalerate dissolved in methanol is used.

**Revendications**

1. Procédé de préparation d'ε-caprolactame par réaction d'esters d'acide 5-formylvalérianique avec de l'ammoniac en excès et de l'hydrogène, en présence de catalyseurs d'hydrogénation et avec utilisation simultanée d'un solvant, à température élevée et sous pression élevée en phase liquide, caractérisé en ce que
   a) on fait réagir l'ester d'acide 5-formylvalérianique avec de l'ammoniac en excès, avec utilisation d'alcanols en $C_1$-$C_4$ en tant que solvants, en présence de catalyseurs au cobalt ou au nickel et sous une pression partielle d'hydrogène de 5 à 1000 bar à une température de 40 a 130°C,
   b) on élimine, du mélange réactionnel obtenu, l'ammoniac jusqu'a une teneur de 0,1 à 2% en poids et l'hydrogène, et
   c) on chauffe le mélange réactionnel ainsi obtenu à une température de 150 a 250°C, puis on récupère le caprolactame.

2. Procédé selon la revendication 1, caractérisé en ce qu'on utilise des catalyseurs qui ont été obtenus par calcination de composés de formule I

$$[(Mg_aNi(II)_bCo(II)_c)Al_2]CO_3(OH)_{16} \times 4\ H_2O$$

dans laquelle a est mis pour un nombre entier ou décimal de 0 à 4 et b et c sont mis pour des nombres entiers ou décimaux de 0 à 6, étant spécifié que 2 x (a + b + c) = 12, à des températures de 200 à 600°C, suivie de réduction avec de l'hydrogène à température élevée.

3. Procédé selon la revendication 1, caractérisé en ce qu'on utilise des catalyseurs au nickel qui contiennent de 30 à 60% en poids de nickel sous forme finement divisée, déposé sur du silicate de magnésium.

4. Procédé selon l'une quelconque des revendications 1 à 3, caractérisé en ce qu'on fait passer l'ester d'acide 5-formylvalérianique en solution avec des alcanols et de l'ammoniac sur un catalyseur disposé en lit fixe, suivant le mode opératoire par circulation du bas vers le haut.

5. Procédé selon la revendication 1, caractérisé en ce qu'on utilise du nickel de Raney ou du cobalt de Raney, suivant le mode opératoire en suspension.

6. Procédé selon les revendications 1 et 5, caractérisé en ce que le nickel de Raney ou le cobalt de Raney est fixé magnétiquement ou électromagnétiquement dans la zone de réaction.

7. Procédé selon l'une quelconque des revendications 1 à 6, caractérisé en ce qu'on respecte, dans l'étape a, un temps de séjour de 1 à 30 mn.

8. Procédé selon l'une quelconque des revendications 1 à 7, caractérisé en ce qu'on respecte une charge du catalyseur de 0,2 à 2,0 kg d'ester d'acide 5-formylvalérianique par litre de catalyseur et par heure.

9. Procédé selon l'une quelconque des revendications 1 à 8, caractérisé en ce qu'on utilise de l'ester méthylique d'acide 5-formylvalérianique en solution dans du méthanol.